# EUROPEAN PATENT APPLICATION

(11) **EP 3 536 286 A1**
(43) Date of publication of application: **11.09.2019**
(21) Application number: 18787702.2
(22) Date of filing: 20.04.2018
(51) Int. Cl.: A61F 5/02, A45F 3/04

(54) **POSTURE CORRECTOR AND RUCKSACK WITH POSTURE CORRECTOR**

(30) Priority: 21.04.2017 ES 201730467 U
(71) Applicant: RM Urgecamino, S.L., 35008 Las Palmas de Gran Canaria (ES)
(72) Inventor: CASTRO LEON, Rafael, 35008 Las Palmas de Gran Canaria (ES)
(74) Representative: Espiell Gomez, Ignacio
(86) International application number: PCT/ES2018/070322
(87) International publication number: WO 2018/193148

(57) **Abstract**

Posture corrector device (1) that is arranged on the lumbar region and that comprises a generally U-shaped stiffening inner structure (2) and that remains housed within an external cover (3) that possesses wings (5) at its end. Preferably the wings (5) are foldable with respect to the central part of the device by means of folding lines (6) and the inner face of the wings possesses a pad (4) to rest the forearms. Also, the object of the invention is a rucksack that comprises a foldable posture corrector device (1) determined by the existence of respective foldable wings (5) on both sides of the rear part (11a) of the bag (11) to support the forearms and forcing the user to straighten his back when he is bearing the rucksack (10) hanging on his back.

## Description

### OBJECT OF THE INVENTION

The invention, as stated in the title of this specification, refers to a posture corrector device and to a rucksack with the incorporated posture corrector providing, to the function to which it is designed, with advantages and characteristics that are disclosed in detail thereafter and that mean a significant novelty in the current state-of-the-art.

The object of this invention refers to a posture corrector, namely of the posture of the back, in order to provoke that the back is in straight position and not adopting a hunched position.

The posture corrector is characterized by its special configuration and the design of the device that, combined with a correct placement on the back, allows to correct, in a simple manner and almost without effort, the position of the back, avoiding to adopt hunched positions.

An additional object of the invention is a conventional rucksack, of those designed to carry objects by means of a bag provided with straps hanging on the shoulders to be borne on the back, that is distinguished in that it includes, in addition, incorporated to the said bag, a foldable posture corrector device that allows, in a simple and easy manner and at will, forcing the user to straighten his back when he is bearing the rucksack hanging on his back, avoiding to adopt hunched postures.

### FIELD OF APPLICATION OF THE INVENTION

The field of application of this invention is within the sector of the industry engaged in the production of devices that, placed on the body, seek to correct the posture position of the back, as well as within the sector of the industry engaged in the production of rucksacks.

### BACKGROUND OF THE INVENTION

Currently, multiple orthopedic devices exist in the market, designed to maintain the back straight, most of them based in support-type structures, as well as multiple models of rucksack, and also some can exist matching both, because the use of rucksacks, already on its own, can arrive to mean an overstrain for the spine, they are usually devices that are complex in their production and design that, in addition, result uncomfortable in their use, their placing or withdrawing being not easy, because generally they provide the existence of straps or corsets surrounding the abdomen.

The objective of this invention is, therefore, to develop a posture corrector easy to produce and use, as well as providing a rucksack that, in addition to its conventional use to carry objects, can constitute at the same time a practical and comfortable means of posture correction and that avoids the drawbacks that are produced and, therefore, is easy as well to produce as to bear, allowing its use in a simple manner, that means, as a conventional rucksack and, when it is wished, also its use as posture corrector.

On the other hand, and as a reference to the current state-of-the-art, it shall be pointed out that the existence is unknown of a posture corrector or of any rucksack with posture corrector, or another invention having a similar application, possessing technical and structural characteristics equal or similar to those shown by that that are herein claimed.

### EXPLANATION OF THE INVENTION

The object of this invention is a device, posture corrector of position of the back, that makes that the user adopts a straight position and walks or is standing with a straight back.

Also, the object of the invention, in a complementary manner, is to endow the said device with means for the massage of the back, so that, simultaneously to achieving maintaining the back in straight position, it is achieved to massage the lumbar region in a simple manner.

The device comprises a U-shaped inner stiffening structure that shows a central region and ends arranged so that the central region defines a recess or concavity with respect to the ends that has dimensions such that it allows to house the back.

The said U-shaped inner stiffening structure remains housed within an external cover, preferably made of a breathable material, for example textiles, and that possesses wings that optionally and complementarily can be foldable with relation to the centre in order to be able to reduce the dimensions of the final device.

The wings of the external cover by the inner face or face that remains in contact with the forearms possess pads in order to facilitate resting the arms so that when exerting a slight force with the forearms forwards, unintentionally, it occurs that the back remains in straight position.

Also, and complementarily to the device, it can possess embossed half-spheres that would be situated where the device is supported on the lumbar region, so that, when walking with the device, they manage to massing the lumbar region.

The object of the invention is as well the posture corrector as the rucksack with posture corrector, all being configured therefore as a significant novelty within its field of application, because when implementing it, the above-mentioned objectives are reached, the characterizing details making it possible and distinguishing it being duly included in the final claims attached to this description.

Concretely, what the invention proposes, as it was mentioned before, is a rucksack designed to carry objects to be borne on the back, that is structured in a conventional manner out of a bag with, at least, two straps hanging from the shoulders, distinguished because it includes, incorporated in the said bag, a foldable posture corrector device that allows, in a simple and easy manner and at will, forcing the user to straighten his back when he is bearing the rucksack hanging on his back, avoiding to adopt hunched postures as well if he is walking bearing it as if he is not or only when he is standing.

For this, on both sides of the rear part of the bag, that means, the part that remains in contact with the back of the user, when it is hanging on his back, the existence of respective foldable wings is provided that, in an unfolded position of use, emerge extending laterally on both sides of the bag, determining respective supporting surfaces for the forearms of the user and, in a folded position of non-use they remain gathered up without projecting from the bag in order not to be a hindrance to the normal use of the rucksack, preferably folded on the sides of the bag.

Advantageously, both wings, that are located at the same height and are symmetrical, possess a slightly curved configuration at the end proximal to them, that means, that joins it to the bag, so that the supporting surface for the forearms remains on a plane more ahead than that of the rear part of the bag that is supported on the back of the user, making its use easier.

Preferably, the wings are arranged at the lower end of the said rear part of the bag, to remain coincident with the lumbar region of the user, although optionally, the said arrangement can be at mid height or at the upper end of the rear part of the bag. It is foreseen that the arrangement of the posture corrector device is fixed with respect to the rear part of the bag or adjustable in height thanks to means allowing to regulate its height with respect to said the upper part of the bag, so that they can be adapted to the requirements and height of each user.

The user, apart from being able to support his forearms on the posture corrector device, can also support his biceps when the posture corrector device is located in an upper position with respect to the rear part of the bag. This circumstance allows to bear heavy rucksacks in a more convenient manner.

Anyway, the posture corrector device incorporating the bag of the rucksack, although preferably is a fixed device, optionally can be extractable, in order to be able to incorporate it, at will, for example inserting it through a given housing between a double layer provided to that effect at the rear part of the bag.

The disclosed rucksack with posture corrector consists, therefore, of an innovating structure having characteristics unknown until now for the aim to which it is designed, reasons that, joined to its practical utility, provide it with sufficient ground to obtain the privilege of exclusivity applied for.

### DESCRIPTION OF THE DRAWINGS

To complement the description that is being made and in order to assist to best understand the characteristics of the invention, attached to this specification, as an integral part thereof, is a set of drawings in which, for illustration and non-limiting purpose, the following has been represented:
The figure 1 shows an individual bearing the posture corrector device object of the invention.
The figure 2 shows in perspective a constructive detail of the device.
The figure 3 shows a possible embodiment of the stiffening inner structure.
The figure 4 shows a second possible embodiment of the stiffening inner structure.

The figures numbers 5 and 6.- They show the views, in rear and front perspective, respectively, of an example of the rucksack with posture corrector object of the invention, both represented with the corrector device in folded position, its configuration and arrangement can be seen.

The figures numbers 7 and 8.- They show rear and front views, of the rucksack appearing in the preceding figures, in this case represented with the posture corrector in position of use; and

The figures numbers 9 to 12.- They show respective rear and front views in perspective and in folded position and of use, of the same example of the rucksack, according to the invention, shown in the four preceding figures, in this case represented with the posture corrector arranged at mid-height, instead of at the lower part of the bag.

### PREFERRED EMBODIMENT OF THE INVENTION

At the sight of the mentioned figures, and according to the numeral adopted, it can be seen in them examples of non-limiting embodiment of the corrector device as well as the rucksack with posture corrector of the invention, which comprises the parts and elements that are mentioned and disclosed in detail thereafter.

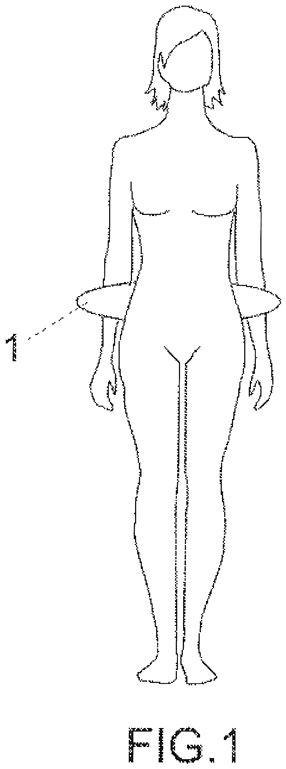

In the figure 1 a front view can be seen of an individual bearing the posture corrector device (1) object of the invention, that as it can be seen it is arranged in the lumbar region, laterally projecting by means of two wings serving as support of the region of the forearms, which provokes the back stretching.

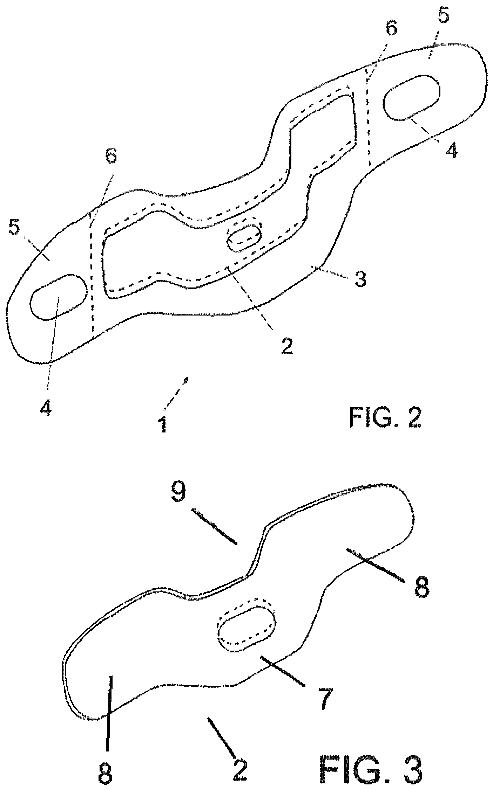

The device as it appears in the figure 2 comprises a stiffening inner structure (2) generally U-shaped and that remains housed within an external cover (3) made of breathable material.

The external cover (3) possesses at its ends wings (5), preferably foldable along a folding line (6) with respect to the central part where the stiffening structure is housed.

In a complementary manner and in order to facilitate the support of the forearms on the internal face of the wings (5), these later possess pads (4) serving for the forearms be supported and at the same time they make that the back remains in vertical position.

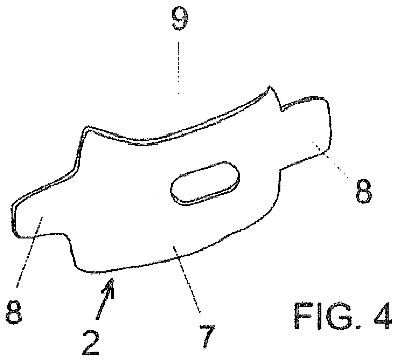

In the figures 3 and 4 two different embodiments of the stiffening inner structure (2) are shown, that in both cases possess a central area (7) and two ends (8) arranged on both sides of the central area (7) so that the central area remains recessed with respect to the ends (8) defining a concavity.

The said concavity (9) possesses a width such that it allows housing the width of the back in the lumbar region, and a depth on the order of 5 cm, so that the wings (8) of the device remain slightly ahead with respect to the central area and thus serve as support to the forearms, making that these later when slightly pulling back produce the posture correction leaving the back in straight position.

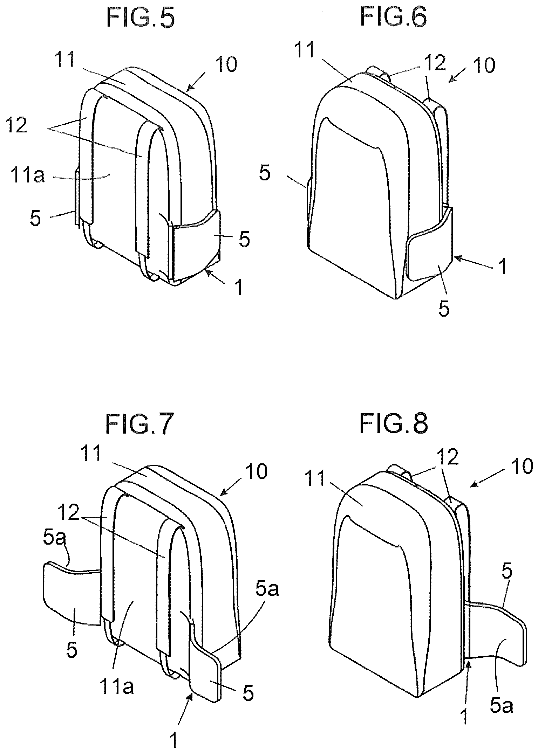

On its hand, as it can be seen in the figures 5 to 12, the rucksack (10) of the invention, as it is constituted in a known manner out of a bag (11) with, at least, two hanging straps (12) to be borne on the back, has the peculiarity that it includes, incorporated in the said bag (11), a foldable posture corrector device (1) the use of which allows forcing the user to straighten the back when he is bearing the rucksack (10) hanging on his back, which is determined by the existence, on both sides of the rear part (11a) of the bag (11), of respective foldable wings (5), which have an unfolded position of use, in which they are emerging extending laterally on both sides of the bag (11) determining respective supporting surfaces (5a) for the forearms of the user, as it can be seen in the figures 7, 8 and 11, 12, and a folded position of non-use, that can be seen in the figures 5, 6 and 9, 10, where they remain gathered without protruding from the bag (11), preferably folded on the sides thereof.

Preferably the two wings (5) forming the foldable posture corrector device (1) are located at the same height with respect to the lower end of the bag (11) and they are symmetrical.

The two mentioned wings (5) constituting the foldable posture corrector device (1) have a slightly curved configuration at the end proximal to them, where they are joined to the bag (11), that locates the supporting surface (5a) for the forearms on a plane more ahead than that of the rear part (11a) of the bag (11) that is supported on the back of the user.

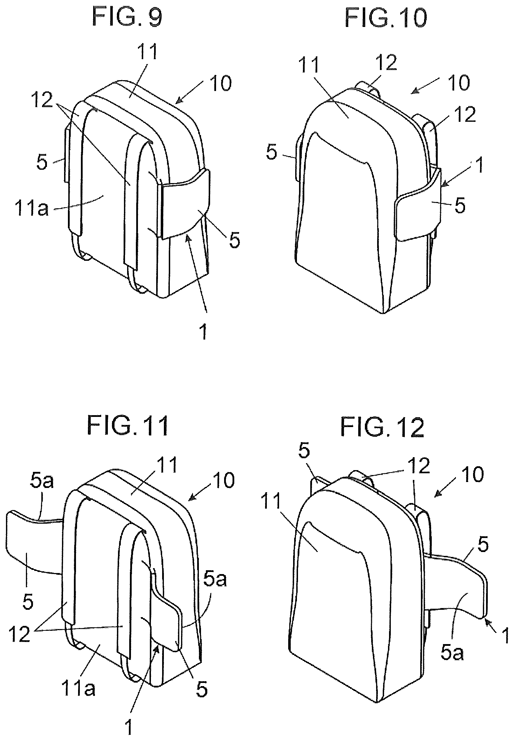

Conveniently, the wings (5) are arranged located at the lower end of the rear part (11a) of the bag (11), as it is shown in the example represented in the figures 5 to 8, although, as it is shown in the figures 9 to 12, in an alternative option, the wings (5) are arranged at mid-height of the said rear part (11a) of the bag (11). In a variant of embodiment, the wings (5) are adjustable in height.

Anyway, the foldable posture corrector device (1), of the invention in an option of embodiment, is a fixed device been joined integral with the bag (11) and, in a variant of alternative embodiment, it is an extractable device for which, for example, it is constituted by a single part, substantially flat and elongated, the side ends of which determine the respective wings (5) with supporting surface (5a) for the forearms of the user, the said part being insertable through a housing provided for that purpose in the rear part (11a) of the bag (11).

The nature of this invention being sufficiently disclosed, as well as the manner to implement it, it is not deemed necessary to extend its explanation any longer in order that any person skilled in the art understands its scope and the advantages arising from it, and it is stated that , within its essence, it can be implemented with other embodiments that differ in detail from the mentioned for example purpose and that will also be covered by the protection that is applied for, provided that its main principle is not altered, changed or modified.

## Claims

1. Posture corrector device (1) **characterized in that** it is arranged on the lumbar region and comprises a stiffening inner structure (2) being generally U-shaped and that remains housed within an external cover (3) that possesses wings (5) at its end.

2. Posture corrector device (1) according to the claim 1 **characterized in that** the wings (5) are foldable with respect to the central part of the device by means of folding lines (6).

3. Posture corrector device (1) according to the claim 1 **characterized in that** on the inner face of the wings (5), there are pads (4) for resting the forearms.

4. Posture corrector device (1) according to any of the preceding claims **characterized in that** the device (1) comprises embossed half-spheres that are located where the device is supported on the lumbar region.

5. Posture corrector device (1) according to any of the preceding claims **characterized in that** the stiffening inner structure (2) has a central area (7) and two ends (8) arranged on both sides of the central area (7) so that the central area remains recessed with respect to the ends (8) defining a concavity (9).

6. Posture corrector device (1) according to the claim 5 **characterized in that** the concavity (9) has a width such that it allows to house the width of the back in the lumbar region.

7. Posture corrector device (1) according to any of the preceding claims **characterized in that** the external cover (2) is made of a breathable material.

8. Rucksack with posture corrector that, being constituted out of a bag (11) with, at least, two hanging straps (12), to be borne on the back, is **characterized in that** it comprises, incorporated in the said bag (11), a foldable posture corrector device (1) determined by the existence of respective wings (5), foldable at both sides of the rear part (11a) of the bag (11) to support the forearms and forcing the user to straighten the back when he bears the rucksack (10) hanging on his back.

9. Rucksack with posture corrector, according to the claim 1, **characterized in that** the wings (5) have an unfolded position of use, which emerge laterally extending on both sides of the bag (11) determining respective supporting surfaces (5a) for the forearms of the user, and a folded position of non-use, in which they remain gathered without protruding from the bag (11).

10. Rucksack with posture corrector, according to the claim 2, **characterized in that** the wings (5), in their folded position of non-use, remain folded on the sides of the bag (11).

11. Rucksack with posture corrector, according to any of the claims 1 to 3, **characterized in that** the two wings (5) forming the foldable posture corrector device (1) are located at the same height with respect to the lower end of the bag (11) and are symmetrical.

12. Rucksack with posture corrector, according to any of the claims 1 to 4, **characterized in that** the two wings (5) constituting the foldable posture corrector device (1) have a slightly curved configuration at the end proximal to them, where they are joined to the bag (11), that locates the support for the forearms on a plane more ahead than that of the rear part (11a) of the said bag (11).

13. Rucksack with posture corrector, according to any of the claims 1 to 5, **characterized in that** the wings (5) are arranged and located at the lower end of the rear part (11a) of the bag (11),

14. Rucksack with posture corrector, according to any of the claims 1 to 6, **characterized in that** the wings (5) appear located at mid-height of the rear part (11a) of the bag (11).

15. Rucksack with posture corrector, according to any of the claims 1 to 7, **characterized in that** the wings (5) are adjustable in height.

16. Rucksack with posture corrector, according to any of the claims 1 to 8, **characterized in that** the foldable posture corrector device (1) is a fixed device, being joined integral with the bag (11).

17. Rucksack with posture corrector, according to any of the claims 1, 4, 5 and 9, the claim (1) (sic) **characterized in that** the foldable posture corrector device (1) is an extractable device.

18. Rucksack with posture corrector, according to any of the claims 1,4, 5, 9, 10 or 11 **characterized in that** the foldable posture corrector device (1) is constituted by a single part, substantially flat and elongated, the side ends of which determine the respective supporting wings (5) for the forearms of the user, the said part being insertable through a housing provided to that purpose on the rear part (11a) of the bag (11).
